Europäisches Patentamt

European Patent Office

Office européen des brevets

(19)

(11) Publication number: **0 205 337**

**A2**

(12) **EUROPEAN PATENT APPLICATION**

(21) Application number: **86304402.0**

(22) Date of filing: **10.06.86**

(51) Int. Cl.⁴: **A 61 K 39/00**
**A 61 K 39/395, C 12 P 21/00**
**//G01N33/577**

(30) Priority: **11.06.85 US 743575**
**09.09.85 US 774057**

(43) Date of publication of application:
**17.12.86 Bulletin 86/51**

(84) Designated Contracting States:
**AT BE CH DE FR GB IT LI LU NL SE**

(71) Applicant: **COLLAGEN CORPORATION**
**2500 Faber Place**
**Palo Alto, California 94303(US)**

(72) Inventor: **Ellingsworth, Larry R.**
**3716 Springbrook Avenue**
**San Jose California 95148(US)**

(72) Inventor: **Rosen, David M.**
**3655 Shadyhollow Court**
**San Jose California 95148(US)**

(74) Representative: **Harrison, David Christopher et al,**
**MEWBURN ELLIS & CO 2/3 Cursitor Street**
**London EC4A 1BQ(GB)**

(54) Chondrocyte antigens and monoclonal antibodies.

(57) Two previously unknown antigens associated with endochondral bone growth are disclosed. Preparations of monoclonal antibodies reactive against such antigens and hybridomas capable of their production are also disclosed.

EP 0 205 337 A2

-1-

# CHONDROCYTE ANTIGENS AND
# MONOCLONAL ANTIBODIES

## Technical Field

The invention relates to diagnosis and treatment of conditions associated with endochondral bone growth and to the preparation of monoclonal antibodies useful therein. More specifically, the invention relates to definition of antigens present in cartilage cells and to monoclonal antibody preparations specific for these antigens.

## Background Art

Formation of new bone occurs through two major mechanisms. In the mature individual, "intramembranous" or direct bone replacement predominates except in long bone fractures which repair by the endochondral route. Direct bone formation is characterized by direct differentiation of precursor osteoblasts from the bone marrow into bone cells and multiplication of already committed osteoblasts. New bone formation is continuous and is balanced by resorption in the healthy adult.

On the other hand, in the developing mesenchymal tissue of the embryo, or in the immature individual, the majority of bone formation is bone growth and occurs in the epiphyseal plate at the end of each bone. Such bone growth is "endochondral"--i.e., occurs with the intermediation of cartilage. The cartilage cells (chondrocytes) mediate the formation of a proteoglycan cartilage matrix, which will eventually be replaced by the mineral matrix of bone. The capacity of these chondrocytes to "hypertrophy" and orchestrate the replacement of cartilage by bone mineral characterizes the chondrocytes of this type of cartilagenous tissue and distinguishes them from the chondrocytes present in the stable articular cartilage which occurs in the joints as a permanent structure.

The biochemical details of endochondral bone growth are understood only dimly at present. Briefly, an epiphyseal plate at each end of an elongated bone is the site of this growth. Precursor cells from blood, bone marrow, or muscle enter the plate and are differentiated through mediation of a variety of growth factors including, for example, a chondrogenic factor characterized by Seyedin, et al (U.S. Patent 4,434,094; U.S. serial nos. 630,938 and 705,479) and are first differentiated into chondrocytes. In accordance with the scenario set forth above, these chondrocytes are associated with the formation of a cartilage matrix, but eventually induce mineralization of the surrounding area. The cartilage matrix is remodeled and gradually populated by bone forming osteoblasts. The result is the addition of new bone material between the existing elongated bone and the enlarged terminus.

Little is known about the nature of the chondrocytes and the changes they undergo in this process. The ability of a chondrogenic factor, a 10-30 kD protein, to stimulate the production of cartilage proteoglycan and type II (cartilage) collagen _in vitro_ from fetal rat mesenchymal cells was shown by Seyedin (supra).

Antibodies to bone-related antigens have been previously prepared. Stenner, D.D., et al, _Proc Natl Acad Sci (USA)_ (1984) 81:2868-2872, prepared monoclonal

-3-

antibodies against bone Gla protein and osteonectin, proteins specific to bone. The antibodies were obtained by immunizing mice with an unfractionated extract from bone made under non-denaturing conditions. Caterson, B., et al, J Invest Dermatol (1982) 79:45-50 report production of antisera and monoclonal antibodies against various cartilage proteoglycan components. Others have, thus, explored the nature of the non-collagenous protein and proteoglycan antigens in bone and even prepared antibodies against some of them, without emergence of a coherent pattern.

In addition, certain proteins have been shown to be directly associated with bone. Poole, A., et al, J Cell Biol (1984) 98:54-65, reported the activity of chondrocalcin, a 70 kD protein which has an affinity for hydroxyapatite and which is associated with the calcification of cartilage in endochondral bone formation. Upon reduction, chondrocalcin has a molecular weight of 35 kD (Choi, H.L., et al, J Biol Chem (1983) 258:655-661). Sasse, J., et al, J Cell Biol (1984) 99:1856-1866, used a monoclonal antibody preparation prepared against muscle cell antigens to show that chick embryonic chondrocyte precursors could be separated from muscle cell precursors.

Various disorders of the bone formation process include osteogenesis imperfecta, osteopetrosis, osteoarthritis, and arthritis. There are also a variety of chondrodysplasias or disorders of cartilage formation that affect endochondral bone formation. These conditions are difficult to diagnose at early stages and the progress of therapeutic regimens is difficult to evaluate. Deformation of the skeletal structure during fetal development, for example, can be caused by a number of teratogenic agents, a problem which is

-4-

becoming increasingly common as the number of unfamiliar
chemicals in the environment increases. The incidence
of bone related cancers is highest among children and
presumably results from aberrations in the epiphyseal
bone formation system.

Accordingly, there is a need for reagents and
methods to characterize normal and aberrant epiphyseal
bone growth so as to diagnose and treat conditions
related to cartilage and bone development.

Disclosure of the Invention

The invention provides novel monoclonal
antibody (Mab) preparations which are useful in
evaluating the progress, nature, and treatment of
endochondral bone and cartilage-associated diseases such
as osteopetrosis and osteoarthritis. The antibodies are
specific with respect to antigenic substances associated
with chondrocytes. These antigens are previously
unknown glycoproteins which characterize chondrocytic
cell surfaces during bone growth.

Accordingly, in one aspect, the invention is
directed to a method to produce a monoclonal antibody
preparation specific against the novel antigen Chon22,
to the antibodies so produced, and to this antigen.

In another aspect, the invention relates to a
method to produce a monoclonal antibody preparation
specific against the novel antigen Chon12/18/24, to the
antibody so produced, and to the antigen itself. In
other aspects, the invention relates to hybridoma cell
lines capable of producing the Mabs of the invention, to
methods of producing the Mabs using these hybridomas,
and to methods of diagnosing epiphyseal bone growth
abnormalities using the Mabs of the invention.

Brief Description of the Drawings

Figure 1 shows immunopanning of Chon 12/18/24 and Chon 22 antigens from a partially purified I-125 labeled bone extract.

Figure 2 shows two dimensional immunoblot showing antibody (1A2-20) reactivity with the Chon 12/18/24 antigens.

Figure 3 shows two dimensional immunoblot showing antibody (2A9-28) reactivity with the Chon 22 antigen.

Figure 4 shows immunoperoxidase staining of adult rat articular cartilage with the 1A2-20 antibody. Labeling (brown) is associated with mineralizing cartilage.

Figure 5 shows immunoperoxidase staining of adult rat articular cartilage with the 2A9-28 antibody. Labeling (brown) is cytoplasmically associated with the articular chondrocytes.

Figure 6 shows immunoperoxidase staining of rat epiphyseal cartilage with the 1A2-20 monoclonal antibody; the label is associated with mineralized cartilage and hypertrophied chondrocytes (arrows).

Figure 7 shows immunoperoxidase staining of rat epiphyseal cartilage with the 2A9-28 monoclonal antibody; the label is cytoplasmically associated with hypertrophied chondrocytes (arrows). There was no matrix associated staining.

Figure 8 shows immunoperoxidase staining of fetal (19 days gestation) rat femur with (a) normal mouse sera; (b) sera from immunized mice; (c) supernatant from 1A2-20; (d) supernatant from 2A9-28.

-6-

## Modes of Carrying Out the Invention

The general process used in the invention to prepare desired hybridomas which secrete useful Mabs is as follows: A preparation containing the antigen is used to immunize subject animals to obtain a source of lymphocytes which are capable of secreting desired antibodies. Ordinarily, the subject animal, such as a rat or mouse or larger mammal, is injected intraperitoneally or intravenously with the antigen preparation, usually in conjunction with an adjuvant, and permitted to mount an immune response. Suitable lymphocytes are obtained either from the circulatory system (peripheral blood lymphocytes, i.e., PBLs) or, if the animal can be sacrificed, from the spleen. The lymphocytes contained in the spleen cells or PBLs are then used in the fusion procedure to obtain hybridomas.

In a typical fusion, the lymphocyte preparation is mixed with a culture of an immortalizing cell line, usually from the same species as the immunized animal such as, where appropriate, a murine or rat myeloma. However, other methods of immortalization are possible, such as transformation with an immortalizing virus such as the Epstein-Barr virus. In the fusion, the preparations of antibody-producing lymphocytes and immortalizing cells are mixed in the presence of a fusing agent, most commonly polyethylene glycol, and plated out into a selective medium. The most commonly used selective medium is the hypoxanthine-aminopterin-thymidine (HAT) medium, which permits survival of only those cells containing a bypass pathway for synthesis of nucleic acids, which most lymphocytes have but most immortalizing cell lines lack. Therefore, in the HAT medium the immortalizing myeloma cell lines will die (due to their lack of the bypass); unhybridized lymphocyte cell lines will die simply because they are not immortalized. Only fused hybridomas will survive, as they share the common features of immortality and the presence of the bypass pathway. The selection medium needs, of course, to be chosen to correspond to the metabolic characteristics of the immortalizing fusion partner.

Surviving cultures, after a suitable time period on the selection medium, are spread onto microtiter plates and screened for the production of desired antibody. Supernatants from the microtiter wells are tested using any convenient immunoassay for the presence of antibodies against the antigen preparation used in immunizing the mammal or other desired antigen. A variety of such techniques is available, including radioimmunoassay and enzyme-mediated immunoassay techniques. In a typical procedure, microtiter plates are coated with the antigen preparation, washed, incubated with various dilutions of the supernatants to be tested, washed again, and then treated with a labeled antibody prepared against the species of origin of the expected Mabs. Only wells where the Mabs have been retained by the antigen preparation will be labeled by the second antibody. Variations on this method are, of course, possible, and, indeed, common. The screening procedure can be modified and designed to screen for a particular pattern of antibody characteristics.

Suitable hybridoma colonies which are shown to secrete antibodies of desired characteristics are then subcultured by successive transfers until it is clear that a single "monoclonal" cell line is present. Such cultures can then be maintained indefinitely and cultured under suitable conditions to obtain the desired supplies of the Mabs. In addition, the hybridomas may be injected into suitable subjects, most commonly mice or rats, to effect an _in vivo_ culturing of the hybridoma by induction of antibody producing tumors. This results in a high concentration of the antibody in the ascites fluid (a peritoneal exudate) as well as in the bloodstream. Of course, the resulting Mab preparation is not entirely pure, since the indigenous antibodies of the host will also be present. However, these are present only in minimal amounts, and the desired Mab predominates sufficiently to obtain sufficient purity for most purposes.

As used herein, Mab "specific for Chon22" or "specific for Chon12/18/24" refers to Mabs which show immunoreactivity with these specific antigens. However, it is well understood that a particular antigen may have a number of antigenic determinants, and that the Mab may be specific for any particular one of these. Accordingly, it is likely that there are a variety of monoclonal antibody preparations which are specific for these antigens, which may differ from each other in the nature of the determinant with which the Mab reacts. Thus, the population of Mabs which are specific for, for example, Chon22, is made up of a number of subsets of "equivalent" Mabs which are specific for the same determinant of Chon22. The equivalents of the Mabs in this subset may be ascertained by cross-reactivity; that is, equivalent Mabs are capable of competing with, and blocking, the immunoreaction of their equivalents with the antigen. Accordingly, equivalent anti-Chon22 Mabs may be shown by labeling one preparation and measuring its ability to bind the antigen in the presence and absence of the other Mab. Equivalent Mab preparations will diminish the quantity of label bound to the antigen.

It is further understood that the constant regions of the heavy-chain portions of the Mabs of the invention will subclassify into the established IgA, IgD, IgE, IgG, and IgM subclasses. A particular monoclonal antibody preparation may be so subclassified. In addition, even equivalent Mabs of the same subclass may differ in primary amino-acid sequence. All of these variations are included in the definition of Mabs specific against Chon22 or Mabs specific against Chon12/18/24.

It is further understood that immunoglobulins, like all other proteins, exist in various ionization states, depending on the surroundings, and may have supplementary molecules associated with them. Immunoglobulins, in particular, are commonly highly glycosylated. The Mabs of the invention include all variations of this sort, so long as they remain functional as defined by their reactivity with the specified antigen.

"Cells" or "cell line" refers to the clearly denoted cells as well as their progeny. It is well understood that the progeny of a particular parent may not be precisely genetically identical, even though reproduction is asexual. The mutation rate is sufficient that minor variations in genetic composition can occur. Therefore, these terms include the progeny of the original cells which are functionally equivalent to their parents, although they may not be precisely identical.

"Immortalizing cell line" refers to a cell line which may be maintained perpetually in culture and is capable of conferring this ability on a cell to which it is fused. Most such immortalizing cell lines are tumor cells, although other means of immortalization can

-10-

occur.  Suitable immortalizing cells lines for the Mabs ,of the invention are typified by the mouse myeloma line P3XAg8653 or the murine line SP2/0 exemplified below.

The antigen preparation used in preparing the monoclonal antibodies is of central importance.  It is prepared from demineralized bone and contains a preponderance of low molecular-weight (below 50 kD) protein, which is bound to cation-exchange resin at slightly acidic pH and in the presence of a chaotropic agent such as urea.  It is believed that such a preparation of preselected protein factors contains proteins important in mediating endochondral bone growth.  As a result of the invention, the nature and *in vivo* location of these factors has been verified.

The example below illustrates the preparation of a suitable immunizing antigen, as well as its use to obtain monoclonal antibody preparations specific against previously unknown Chon22 and Chon12/18/24 antigens. These antigens have been shown by the invention to be significant in the process of *in vivo* endochondral bone growth, and to be present in the cytoplasm of normal chondrocytes, which are responsible for this growth. Accordingly, the Mabs prepared according to the invention and the antigens against which they are specific are instrumental in obtaining normal growth. By assessing the level of these antigens in a subject, it is possible to assess the presence or absence of normal functioning of this mechanism.  Accordingly, the Mabs of the invention are useful in the detection and monitoring of bone diseases.

Examples

The following examples are intended to illustrate but not to limit the invention.

0205337

-11-

## Example 1
## Antibody Secreting Fusion Partner

Female Balb/C mice were immunized with a protein preparation from demineralized bone which was prepared as follows:

Fresh bovine metatarsal bone was frozen on dry ice, cleaned of periosteum and marrow, and pulverized in a liquid nitrogen cooled mill. The pulverized bone was washed overnight in 0.01 M HCl at 4°C and defatted as described by Seyedin, S., et al, *J Cell Biol* (1983) 97:1950-1953. The resulting bone powder was demineralized overnight in 0.5 M HCl and water washed to a pH above 4 to give demineralized bone powder (DMB).

The DMB was extracted with 4 M guanidine/HCl and 1 mM EDTA overnight and then re-extracted for an additional 4 hr, both at 4°C. The extract was concentrated with a Diflo hollow fiber membrane (10 kD MW ultrafiltration system, Amicon Corporation, Danvers, MA) and the concentrate dialyzed against water for 4 days and lyophilized.

The lyophilized material was reconstituted in 4M guanidinium-HCl (pH 6.8) 10 mM EDTA and 0.02% sodium azide and applied to a Sephacryl S-200 (Pharmacia, Piscataway, NJ) molecular sieve column. The fraction of MW < 50 kD was dialyzed against water at 4°C and lyophilized. The lyophilized solid was taken up in a buffer containing 6 M urea, 10 mM NaCl, 1 mM EDTA, and 50 mM sodium acetate (pH 4.8) and applied to a CM-cellulose cation exchange column (Whatman, CM-52) and the bound protein eluted in a 10 mM - 400 mM NaCl gradient. These eluted fractions were pooled, dialyzed against water, and lyophilized.

The pooled fractions were solubilized in 0.01 N HCl at 1-2 mg/ml and mixed with an equal volume of Freund's adjuvant for injection into mice. Female Balb/C mice were given three biweekly intraperitoneal injections of 50 µg extract; the primary injection in complete Freund's adjuvant, the boosters in incomplete Freund's adjuvant.

Three days following the final boost, the mice were bled through the orbital plexus and the serum titrated using an ELISA assay (Example 3A) for antibody to the immunizing extract antigens. Mice with high titers of antibodies were selected and spleen cells prepared as a single cell suspension.

## Example 2

### Preparation of Hybridomas

The spleen cells prepared as in Example 1 were fused with SP2/0 myeloma cells at a 1:1 ratio in polyethylene glycol/1500 by the method of Oi, V., et al, "Immunoglobulin Producing Hybrid Cell Lines" in _Selected Methods in Immunology_ (1980) W.H. Freeman, San Francisco. The fusion mixture was plated into microtiter plates at $10^6$ cells/100 µl. Hybridomas were HAT selected for 10-14 days, and the hybridoma containing cultures assayed for antibody to the antigens prepared in Example 1 using an ELISA assay (see below). Antibody producing hybridomas were cloned and subcloned in soft agar and ascites fluid. Two positive subclones, designated 1A2-20 and 2A9-28 were selected.

-13-

## Example 3

### Characterization of Secreted Mabs

A. Specificity-ELISA Procedure

The selected hybridomas secreted Mabs which were immunoreactive with the extract antigens as assayed by enzyme linked immunosorbent assay (ELISA) described in this paragraph. The assay was used also to screen for high antibody titers in the inoculated mice of Example 1. Verification of this immunoreactivity for the Mabs was, of course, a direct result of the selection procedure.

For the assay, the lyophilized CM-cellulose bound extract obtained in Example 1 was dissolved in 0.01 M HCl at 2 mg/ml and then diluted in 0.02 M carbonate buffer (pH 9.6) to 25 µg/ml. One hundred µl of the buffered solution was added to each well of Falcon PVC 96 well plates and the plates incubated overnight at 4°C. Non-specific protein binding was blocked with PTB buffer (phosphate buffered saline pH 7.2 containing 0.05% Tween 20 and 1% wt/v bovine serum albumin). The plates were washed, and the antibody dilutions added to each well and the plates allowed to incubate for 1 hr. The plates were washed with PT buffer (phosphate buffered saline containing 0.05% Tween 20) and peroxidase-conjugated rabbit $F(ab')_2$ antimouse IgG, IgM, and IgA (Zymed, SSF, CA) was added for a 1-2 hr incubation. The plates were then exhaustively washed, and 100 µl of 2,2'-azino-di-(3-ethylbenzthiazoline) sulfonic acid (ABTS) substrate solution containing 0.03% ABTS, 0.03% $H_2O_2$ in 0.1 M citrate buffer, pH 4.0, was added. The color was allowed to develop for 30 min and the plates read at 414 nm with a Titer Tek Multiscan (Flow Laboratories, San Diego, CA). Culture supernatants and ascites fluid

-14-

prepared from the cell lines of Example 2 were tested at various dilutions. The cell supernatants and ascites fluid from the lines 1A2-20 and 2A9-28 gave high titers. These cell lines were deposited with ATCC on 21 August 1985.

B.  Determination of Antibody Class

The Mabs from the selected hybridomas were classified using immunodiffusion against Ig class specific antisera (Cappel Labs, Cochranville, PA) after purification with either DEAE Affi-gel Blue (Biorad, Richmond, CA) or Sepharose protein A (Pharmacia). The Mabs secreted from 1A2-20 and from 2A9-28 were both classified as IgGl.

C.  Identification of Specific Antigenic Proteins

The particular proteins contained in the pooled eluate from the CM-bound extract of Example 1 which were immunospecific for the Mabs were identified by immunopanning and immunoblotting techniques. Purified Mab from the culture supernatants was suspended in 0.02 M carbonate buffer, pH 4.0, and an amount containing 4 µg Ig added to each well of Falcon PVC microwells and allowed to incubate overnight at 4°C. Non-specific protein binding was blocked with PTB buffer and 1-5 x $10^6$ cpm of $I^{125}$-labeled proteins from the pooled eluate was added for a 2-4 hr incubation. (Labeling of the pooled eluate proteins utilized the lactoperoxidase method of Morrison, M., Methods Enzymol (1980) 70:214-220.)

The plates were exhaustively washed to remove unbound antigens and the immune complexes then dissociated with SDS-sample buffer and directly applied to a 15% SDS polyacrylamide gel, and separated according

-15-

to the method of Laemmli, U., *Nature* (1970) .227:680-685. The developed gels were dried and autoradiographed with Kodak XR-5 film for 24 hr at -70°C.

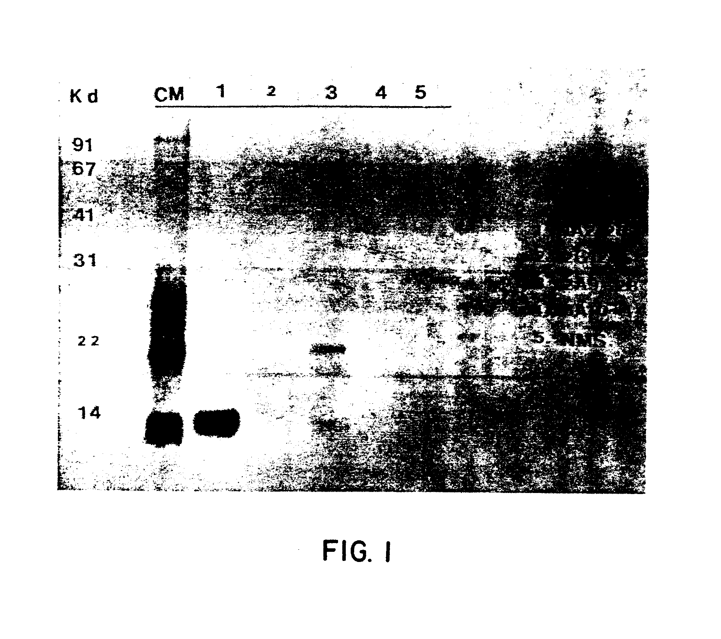

The results are shown in Figure 1. Lane 1 shows that the Mab from 1A2-20 was strongly reactive with a 12 kD protein, and less strongly bound to 18 kD and 24 kD protein bands. Lane 3 shows that the Mab from 2A9-28 bound specifically to a 22 kD protein and less strongly bound to a 12 kD protein. Lanes 2, 4, and 5 represent controls comprising non-specific monoclonal antibody and normal mouse serum.

In a complementary method to determine the nature of the antigen, immunoblotting following two dimensional isoelectric focusing of the potential antigens was employed. The eluted CM-bound antigens from the pooled eluate of Example 1 were focused in the first dimension with pharmalyte pI 3-10 (Pharmacia) and separated by molecular weight in second dimension (15% SDS-PAGE). The separated proteins were electrophoretically transferred to nitrocellulose (Transblot, Biorad, Richmond, CA) at 175 milliamps for 18 hr in 0.025 M Tris, 0.19 M glycine (pH 8.3) containing 20% (v/v) methanol. Duplicate nitrocellulose filters are either stained for total protein or antibody treated.

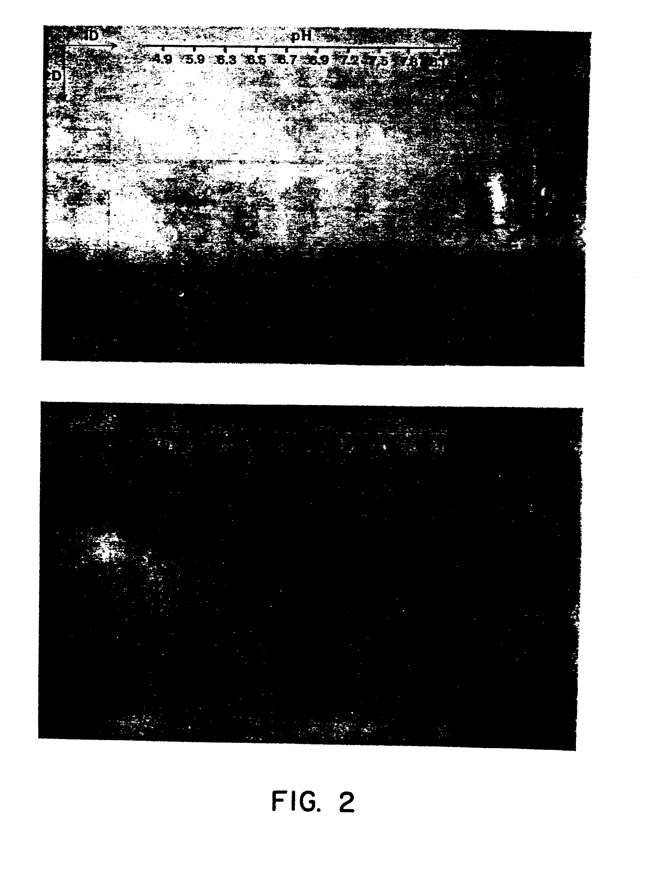

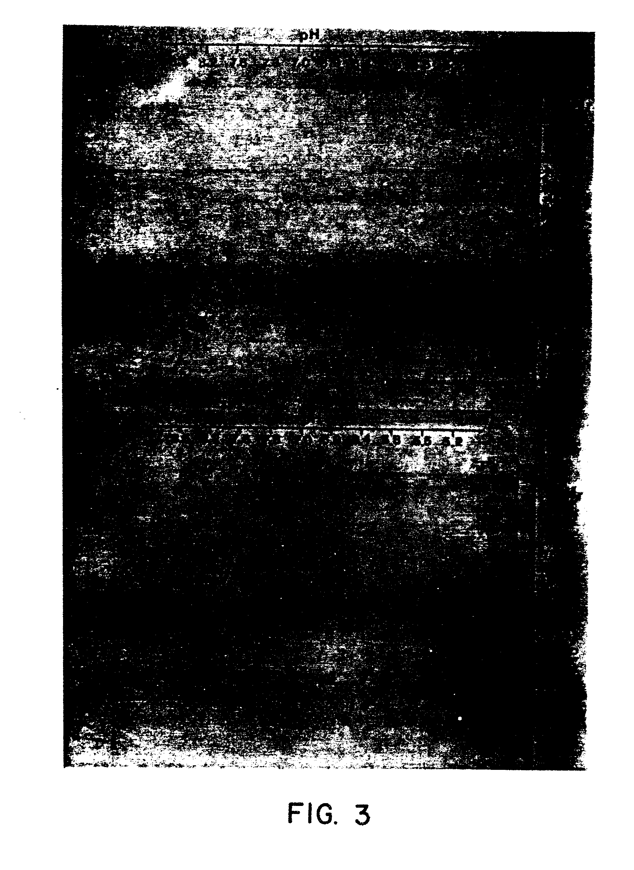

To stain, the nitrocellulose filter was submerged in 0.1% amido black in methanol:acetic acid:water (45:10:45) and destained with the same solution without amido black. Figures 2a and 3a show. filters stained to detect total proteins in the eluates on isoelectric focusing gels using different pH gradients in the first dimension. The eluate appeared

-16-

to contain a mixture of proteins of various molecular weights.

Duplicate filters in each case were antibody treated as follows: non-specific protein binding was blocked with PTB buffer, the filters were washed and then treated with antibody for 2 hr. The antibody-treated filters were washed again and treated with diluted (1:1000) peroxidase conjugated rabbit $F(ab')_2$ antimouse Igs for an additional 2 hr. The blots were then developed with a substrate containing 0.0025% 3,3'-dimethoxybenzidine-dichloride (Sigma), 0.01% $H_2O_2$ in 0.01 M Tris (pH 7.7). The reaction was stopped by washing with water. The results of the antibody binding are shown in Figures 2b and 3b.

As shown in Figure 2b, 1A2-20 Mabs selectively bound proteins of MW 12 kD, 18 kD, and 24 kD having pI values in the range 7.2-8.1. Figures 3b shows that Mab from 2A9-28 binds antigen of molecular weight 22 kD which has 5 distinct isoelectric forms of pI 5.8, 6.0, 6.4, 6.9, and 7.3.

Immunoblots alternatively labeled by treating with [125]I-labeled antimouse Ig (Amersham) confirmed these results.

The antigen proteins associated with 1A2-20 secreted Mabs were designated Chon24/18/22. The antigen proteins corresponding to 2A9-28 Mab were designated Chon22.

D. Localization of Chon22 and Chon12/18/24 in Tissue

The cell and tissue association of the antigens was determined by immunocytochemical staining. Briefly, adult or fetal (19 days) rat femur and the articular cartilage was neutral formalin fixed, decalcified, paraffin embedded, and sectioned by routine methods.

The sections were deparaffinized and endogenous peroxidase blocked with 0.1% peroxide in methanol. These sections were rehydrated in 0.05 M Tris, pH 7.2 saline (15 min) and treated with 1 mg/ml testicular hyaluronidase (Sigma) in 0.1 M sodium acetate, pH 5.5, in saline (30 min at 37°C). Non-specific protein binding was blocked with 0.5% (wt/v) BSA in Tris-saline for 15 min, washed, and the sections were incubated with optimally diluted ascites fluid containing Mab secreted by 1A2-20 or 2A9-28. The sections were then washed with Tris-saline and incubated for 30-60 min with peroxidase conjugated rabbit $F(ab')_2$ antimouse IgG. The slides were treated with diaminobenzidine (DAB) substrate, consisting of 0.5 mg/ml DAB in 0.05 M Tris, pH 7.0, saline containing 0.01% peroxide. The sections were counterstained with Myers hematoxylin (Sigma).

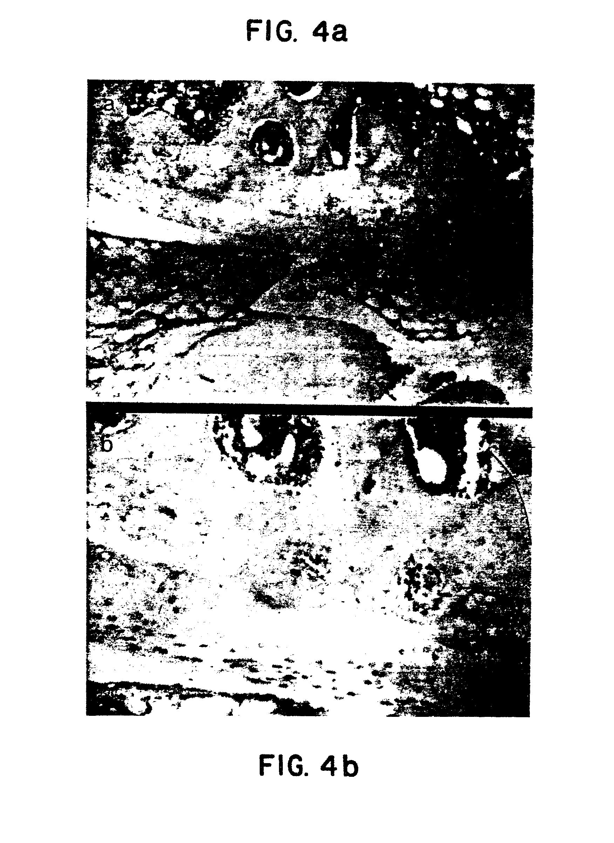

The results are shown in Figures 4-8.

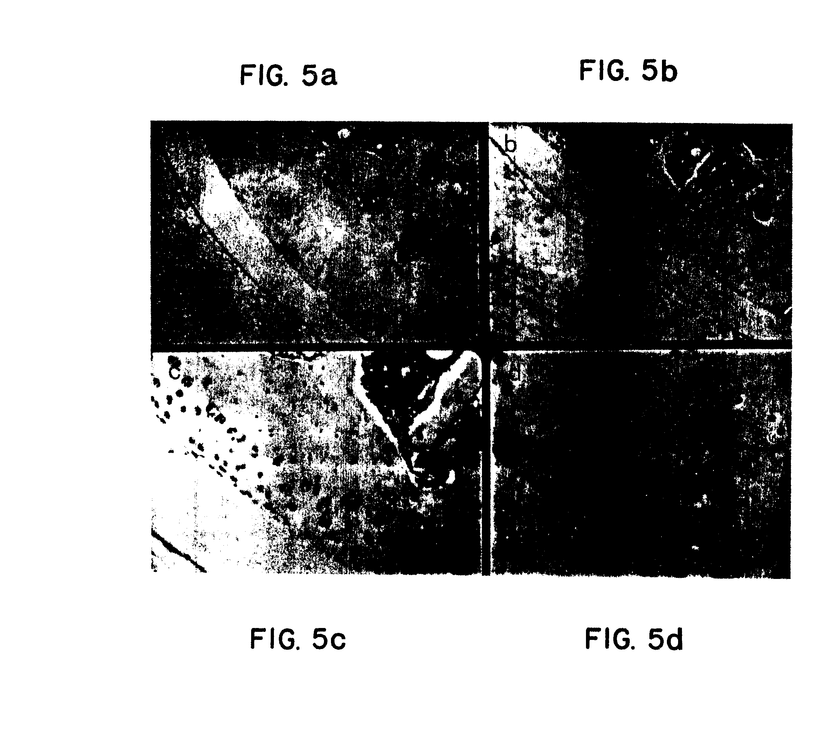

Figures 4a and 4b show articular cartilage labeled with 1A2-20 Mab. The Chon12/18/24 protein was localized at the interface between the articular cartilage and cortical bone, and in the cytoplasm of sparse chondrocytes. Similar results were obtained using bovine articular cartilage. Figures 5a-d show serial sections from the articular cartilage labeled with 2A9-28 Mab showing that the Chon22 antigen was located in the cytoplasm of all articular chondrocytes, but not in the matrix.

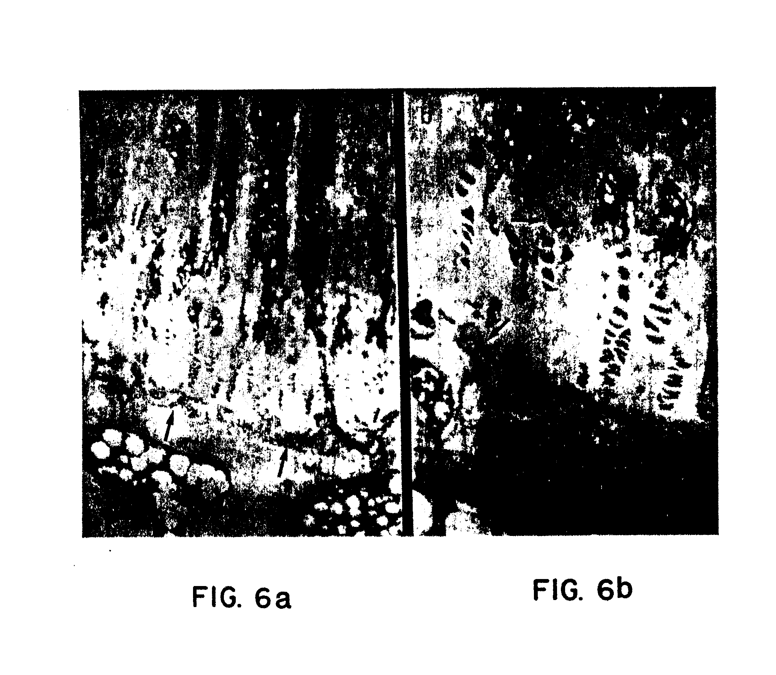

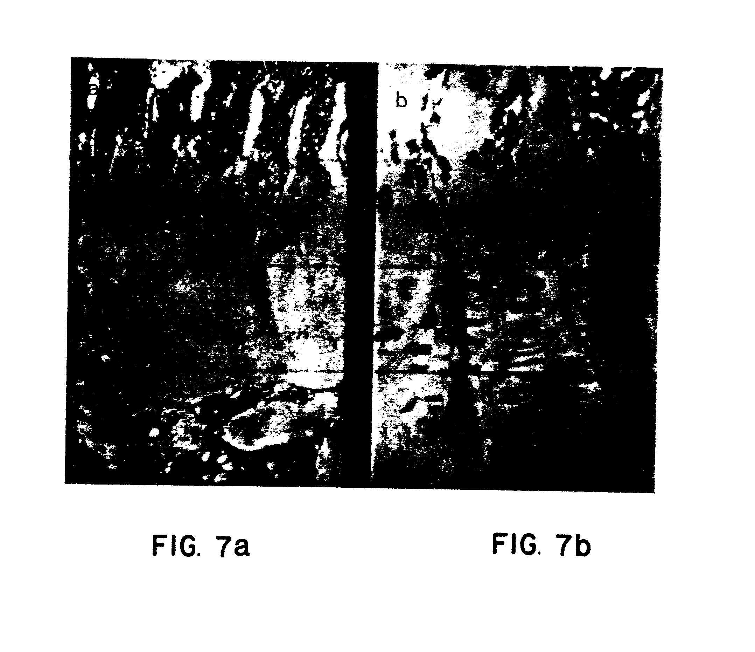

Figures 6 and 7 show the results of labeling the epiphyseal growth plate with 1A2-20 and 2A9-28 monoclonal antibodies respectively. The Chon12/18/24 antigen was localized at the interface between epiphyseal cartilage and cortical bone and within mineralizing epiphyseal cartilage, as well as in the cytoplasm of hypertrophied epiphyseal chondrocytes

-18-

((Figure 6 (arrows)). Figures 7a and 7b show the epiphyseal plate was labeled with 2A9-28 antibody specifically in the cytoplasm of hypertrophied chondrocytes (arrows) but without matrix labeling. Neither Mab was found in the zones of proliferation and maturation.

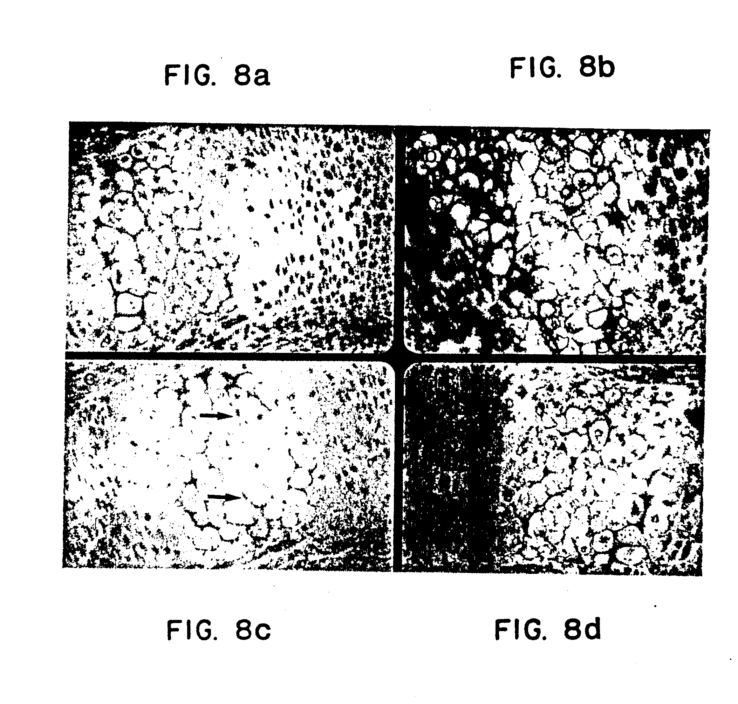

Figures 8a-d show the results of labeling of fetal metatarsal bone with the two Mabs. Figure 8a is a control using normal mouse sera. The metatarsal matrix was found to contain abundant cartilage proteoglycan when labeled with sera of mice immunized with the antigen preparation of Preparation A (Figure 8b). The Chon12/18/24 antigen was found in the matrix surrounding the hypertrophied chondrocytes using the supernatant from 1A2-20 (Figure 8c) within the area of mineralization. In contrast, the Chon22 protein was associated with the cytoplasm of more peripheral chondrocytes using the suprnatant from 2A9-28 (Figure 8d), and there was no matrix staining.

The Chon12/18/24 and Chon22 proteins were never found in sternal cartilage, cortical bone, bone marrow, and soft tissues, e.g., connective tissues, skeletal muscle, liver, and lymphoid organs.

In summary, Chon22 was associated with the cytoplasm of articular chondrocytes and hypertrophied epiphyseal chondrocytes, but not those within the zones of proliferation and maturation. Chon22 was not associated with any matrix material. Chon12/18/24 was present at the interface between articular cartilage and cortical bone, in epiphyseal cartilage and cortical bone and mineralizing epiphyseal cartilage. It was also abundant in fetal endochondral bone.

The cell lines 1A1-20 and 2A9-28 were deposited with the American Type Culture Collection, Rockville, MD, U.S.A. (ATCC), on 21 August 1985. They were assigned ATCC numbers HB 8893 and HB 8892, respectively. These deposits were made under the provisions of the Budapest Treaty on the International Recognition of the Deposit of Microorganisms for the Purposes of Patent Procedure and the Regulations thereunder (Budapest Treaty). This assures maintenance of a viable culture for 30 years from date of deposit. The organisms will be made available by ATCC under the terms of the Budapest Treaty, and subject to an agreement between Applicants and ATCC which assures unrestricted availability upon issuance of the pertinent U.S. patent. Availability of the deposited strains is not to be construed as a license to practice the invention in contravention of the rights granted under the authority of any government in accordance with its patent laws.

-20-

## Claims

1. A preparation of antigens which contains proteins that are markers of cartilage development, prepared by fractionating demineralized bone powder to obtain a fraction containing proteins of molecular weight less than 50 kd, treating said fraction with a cation-exchange resin in the presence of a chaotropic agent at pH about 4-5; and eluting the bound proteins from the resin, and mixing said proteins with an adjuvant.

2. The preparation of claim 1 wherein the demineralized bone powder is prepared by de-fatting pulverized bone to obtain a de-fatted residue and treating said residue with an effective concentration of mineral acid; and wherein

the cation exchange resin is a DEAE resin and the chaotropic agent is 8 M urea.

3. An anti-Chon22 or anti-Chon12/18/24 antibody when produced by immunizing a warm-blooded vertebrate with the preparation of claim 1.

4. The antibody of claim 3 when produced by culturing an immortalized cell line derived from antibody-producing cells of said warm-blooded vertebrate under conditions suitable for antibody production; and recovering the antibody produced.

5. A composition of matter comprising a hybrid continuous cell line which produces antibody to Chon22 or Chon 12/18/24..

-21-

6.    The composition of claim 4 wherein the cell line is a hybrid comprised of a spleen cell from an animal immunized with the preparation of claim 1 fused to a myeloma derived from the same animal species as the spleen cell, and a culture medium for said hybrid.

7.    The composition of claim 6 wherein the hybrid is the cell line 1A2-20 or 2A9-28.

8.    The antigen Chon22 or Chon12/18/24 and antibodies reactive therewith.

9.    Monoclonal antibodies or the equivalents thereof secreted by 1A1-20 or 2A9-28.

0205337

1/8

FIG. I

FIG. 2

FIG. 3

4/8

FIG. 4a

FIG. 4b

FIG. 5a

FIG. 5b

FIG. 5c

FIG. 5d

FIG. 6a                    FIG. 6b

7/8

FIG. 7a          FIG. 7b

FIG. 8a

FIG. 8b

FIG. 8c

FIG. 8d

8/8

0205337